Europäisches Patentamt

European Patent Office

Office européen des brevets

(11) Publication number: **0 479 303 A1**

# EUROPEAN PATENT APPLICATION

(21) Application number: **91116942.3**

(22) Date of filing: **04.10.91**

(51) Int. Cl.⁵: **A61M 5/32**

(30) Priority: **05.10.90 IT 8500290**

(43) Date of publication of application:
**08.04.92 Bulletin 92/15**

(84) Designated Contracting States:
**CH DE ES FR LI**

(71) Applicant: **Morandini, Amorino**
**Corso Garibaldi 51**
**Villafranca, Verona(IT)**

(72) Inventor: **Morandini, Amorino**
**Corso Garibaldi 51**
**Villafranca, Verona(IT)**

(74) Representative: **La Ciura, Salvatore**
**c/o STUDIO D'ORIO Via F. Sforza, 3**
**I-20122 Milan(IT)**

(54) **Accessory mounted on syringes enabling automatic needle withdrawal.**

(57) The present invention is carried out in order to eliminate some inconveniences which can be found in using a conventional syringe whose needle may provoke fortuitous accidents or the diffusion of dangerous illnesses.

Generally, the present device consists of a containing structure (1) which comprises an oblong hollow body (10) for housing and supporting a needle (3). The hollow body (10) is connected to a chamber (15) in which a small piston (4) can run in order to block the needle on the outside for a normal use of the needle. Then, the small piston provokes the needle itself to be withdrawn and encapsulated in an irreversible manner.

Such a withdrawal is caused through the advancing of a push-rod (23) which is moved by a piston of a syringe (2). The small piston (4) and a support (7) of the needle (3) are put in opposition to elastic means (16) and (25) or to attracting and repelling magnetic means.

fig. 2

The present patent of invention refers to a device to be mounted on injection syringes, for instance hypodermic or intravenous injection syringes or the like, also for drawings, for permitting the syringe needle to be withdrawn automatically.

The present invention has been conceived and carried out in order to make the use of the syringes available on the market today safer. The available syringes comprise a piston running inside a cylindrical body at one end of which a needle is mounted. When no safety precaution is taken, such a needle remains uncovered also after it has been used and can be used more times consecutively.

A particular attention is devoted at the present time to the conventional syringes which are very dangerous owing to two main inconveniences:

- the first inconvenience is that the used syringes can be thrown away or also abandoned in whatever place and become the cause of fortuitous accidents on persons who are casually wounded by the uncovered needle;
- the second inconvenience is represented by a possible diffusion of dangerous illnesses as certain persons use the same syringe for injecting drugs into themselves without worrying about a possible contagion of infection or virus which could be even lethal.

The aim of the present invention is to carry out a device which must eliminate the aforecited inconveniences and prevents a syringe, already used for an injection, to be used for other injections. Moreover, such a device makes the eliminating phase of the used syringe safer for the needle is manufactured together with a device which includes the needle itself fully and in an irreversible manner after the needle has been used for an injection.

Within the limits of the above general purpose, the invention proposes a particular kind of retractable needle to be coupled with a conventional syringe. This feature is essential from an economic point of view inasmuch as the safety needles according to the present invention can be used by simply coupling them with the conventional syringes, without the necessity of making important changes in the conventional syringes. It will be sufficient to provide such syringes with simple additional elements which have to co-operate with the needle safety system.

The invention refers to a device to be mounted on injection or drawing syringes or the like for permitting the syringe needle to be withdrawn automatically, characterized in that for comprehending an oblong hollow body for housing and supporting the needle, the hollow body being co-operating with a chamber housing a small piston which can block or release the needle; the chamber being provided with a seat permitting a syringe to be coupled; the small piston being shaped in such way that in a certain position, it can block the needle, once the latter has been pushed out of the hollow body and when the small piston is released backwards in consequence of the advancing of a push-rod operated by a piston of a syringe, the needle is withdrawn and included into the said hollow body, the movement of the said small piston and needle being caused by elastic means or attracting and repelling magnetic means.

The invention will be better understood from the following description, set forth as an example, not limiting the invention as well as from the accompanying drawing in which:

Fig. 1 shows a side schematic view of a device according to the invention, mounted on a syringe and before the use;
Fig. 2 shows a side schematic view of the device according to the invention after the use;
Figs 3 and 4 show schematical views of details comprised in Figs 1 and 2, with the locking and releasing needle means before and after the use respectively.

With reference to the accompanying drawing, number 1 denotes a device to be mounted on a syringe 2 for permitting the needle to be automatically withdrawn according to the invention as a whole.

The device 1 consists of a hollow body which is essentially L-shaped and in which the vertical part contains and supports a needle 3 whereas the horizontal part comprises a small piston 4, particularly shaped, and a vertical seat 5 which is open on the upper part for permitting an end 6 of the syringe 2 to be inserted.

The needle 3 is mounted on a preferably square-sectioned support 7 for preventing possible axial rotations, the said support being provided with a channel 8 shaped in such way that it can penetrate the needle 3 till reaching a side hole.

The support 7 of the needle 3 can run within a chamber 9 of a vertical part 10 which, in the present example, has a square section like the one of the support 7 itself. The needle 3 can get out through a lower hole 11 which is provided at the base of the chamber 9.

Furthermore, the chamber 9 is separated from the horizontal part 12 through a wall 13, provided with a hole 14, which communicates with the chamber 15 of the horizontal part 12 itself.

A small piston 4 can run within the said chamber and comprehends a rubber part 16 which has an essentially square shape and is coupled with a rigid part 17 provided with an upward tooth 18.

When the rubber part 16 is compressed against the partition wall 13, such a tooth can enter a hole 19 communicating with the seat 5.

Moreover, a channel 20 passes through the small piston 4 for its entire length. The channel 20 is fixed to and passes through a rigid part 17 and gets out of the rubber part 16 to a certain extent so as to form an extension 21 which penetrates into a side hole of the support 7, such a hole being formed by the end of the channel 8.

The rubber part is provided with a flanging 22 acting as a stuffing box, which can insert perfectly into the hole 14 and touch the side of the support 7 lightly.

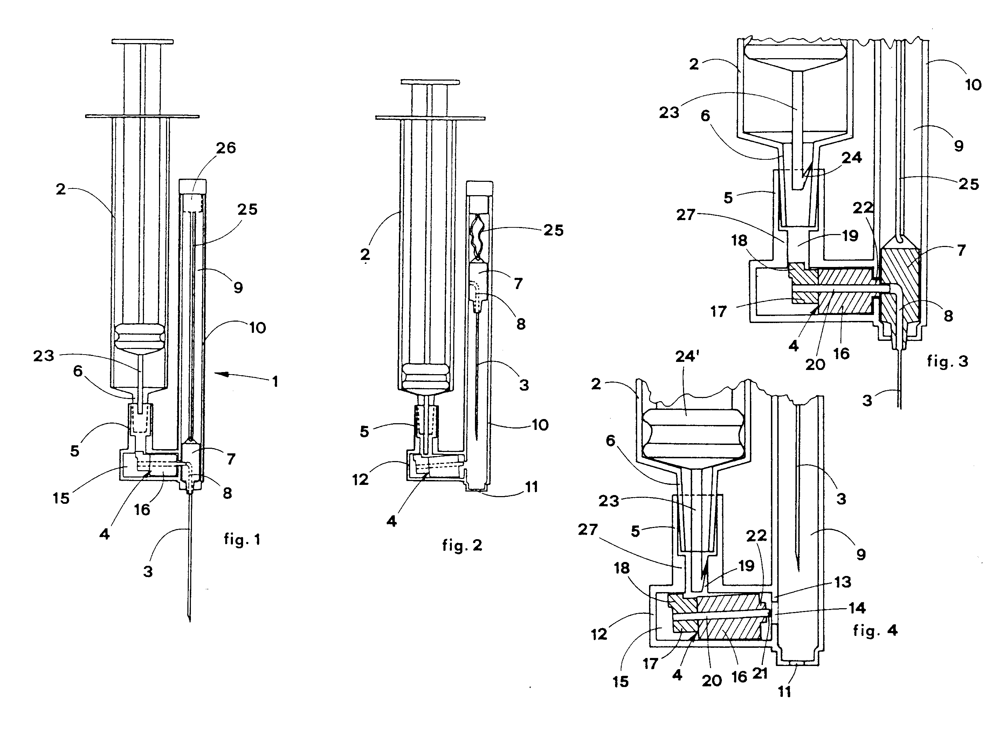

As it can be seen in Fig. 3, when the tooth 18 is inserted in the hole 19, the rubber part 16 of the small piston 4 is compressed against the wall 13 and when the tooth itself gets out of the hole 19, the small piston is released backwards.

In the present example, the means for pushing the tooth 17 downwards is a push-rod or nail 23 to be mounted on a rubber part 24' of the piston of the syringe 2.

The push-rod 23 can penetrate through the syringe end 6 into the seat 5 so that it can enter the hole 19, when the push-rod is in its maximum thrust position, and push the tooth 18 out of the hole itself.

Further, the push-rod 23 can be provided with a hook 24 for preventing the nail and the piston to return back for re-loading the syringe.

Finally, the support 7 of the needle 3 is provided with means which permit the needle itself to be withdrawn into the chamber 9. These means consist of a rubber band or a spring or the like 25, which is connected to the part 7 itself at one end, and to a cap 26 at the other end. The cap 26 is sealed on the upper end of the body 10. According to a possible variant, the means for permitting the needle to be withdrawn can be a magnet or other magnetic element to be mounted on the cap 26 for attracting a metal surface to be provided on the support 7.

Now, we shall describe an example for operating the device according to the present invention.

Before the use, the device 1 appears as it can be seen in Figg. 1 and 3, that is the needle 3 is out of the hole 11 and hold in this position by the extension 21 of the channel 20 which extension penetrates the mouth of the channel 8 when the small piston 4 is pushed against the wall 13 and is locked in this position through the tooth 18 which has entered the opening 19.

In this situation, there are two forces which exercise their thrusts: the rubber element 16 pushes the small piston backwards by making contrast with the wall 13 whereas the rubber band 25 is attracting the support 7 towards the cap 26.

When the piston of the syringe 2 is lowered in order to push the fluid towards the needle 3, the push-rod 23 penetrates the seat 5 and pushes the tooth 18 which lowers and goes out of the hole 19.

In this phase, the liquid to be injected passes through the hole 19 into the chamber 15 and from the chamber 15, the liquid penetrates the needle 3 through the channel 20. The small piston 4 takes up almost all the room of the chamber 15 so as to prevent the liquid to be too much diffused to the detriment of the utilizable quantity.

The backward release of the small piston 4, caused by releasing the tooth 18 and by the thrust of the rubber element 16, permits the extension 21 to get out of the opening of the channel 8, the support 7 being simultaneously let free from the needle which draws back into the inner part of the chamber 10 towards the cap 26 owing to the rubber band 25 or other elements such as a magnet or also an air pocket.

In Figs 2 and 4, the needle device is shown after it has been used. As it can be seen, it is absolutely not possible to re-load the needle for a second use. In fact, on the one hand, it is no more possible to lock the needle out of the chamber 9, and on the other hand a hook 24 prevents the piston of the syringe 2 to draw back.

As an advantage, instead of placing the push-rod 23 in the syringe, that is on the rubber part 24' of the piston, it is possible to place the push-rod 23 in an inner contraction 27 of the part 5 and to proceed then in the same way as described above.

That is very advantageous when it is considered that the device can be used with a conventional syringe, normally available on the market, without making any changes in the syringe itself.

A further advantage is that the channel 20, according to a possible variant, can have the opposite end, in respect of the extension 21, directed upwards in order to form a mouth near the chamber 19. This permits the liquid to directly penetrate the channel 20 without passing through the chamber 15 so as to make the liquid discharge easier and to considerably reduce the space reserved for the passage of the liquid.

## Claims

1. Device to be mounted on syringes for injections, drawings or the like for automatically withdrawing the needle, characterized in that for consisting of a containing structure which comprises an oblong hollow body (10) housing and supporting a needle (3), a needle support (7) being locked or released by a small piston (4) for permitting the needle (3) to be locked or released respectively; the small piston (4) being arranged into a chamber (15) which is placed laterally in respect of the said body (10) and comprehends a seat (5) for permitting a syringe (2) or the like to be coupled; the said small piston (4) being shaped in such a way that it can lock the needle (3), when the latter has been pushed out of the hollow body (10), and the small piston (4) can provoke the withdrawal and the introduction of the needle (3) into the said hollow body (10) when the small piston itself is released backwards in opposition to a rubber element (16), such a release being caused by advancing a push-rod (23) operated by the piston of the syringe (2); the movement of the said small piston (4) and needle (3) being permitted by antagonist elastic means (25) or magnetic means or the like.

2. Device as claimed in claim 1, characterized in that the said support (7) of the needle (3) is passed through by a channel (8) which is connected to the needle itself and ends laterally to the support (7).

3. Device as claimed in the preceding claims, characterized in that the said small piston (4) consists of a rubber part (16) coupled with a rigid part (17) which is provided with an upward tooth (18).

4. Device as claimed in the preceding claims, characterized in that the said rubber part (16) of the small piston is provided with a flanging (22) which can enter the said needle hollow body (10).

5. Device as claimed in the preceding claims, characterized in that the said small piston (4) takes up nearly all the room of the chamber (15) in which it is inserted so as to prevent an excessive dispersion of the liquid to be injected.

6. Device as claimed in the preceding claims, characterized in that the said small piston (4) is passed through by a channel (20) projecting with an extension (21) which can lock the said needle support (7).

7. Device as claimed in the preceding claims, characterized in that the said push-rod (23) can be provided with a hook (24) or the like for preventing the piston of the syringe (2) to go back.

8. Device as claimed in the preceding claims, characterized in that the tooth (18) of the said small piston (4) can penetrate a hole (19) connected with the said seat (5) housing the syringe (2).

fig. 3

fig. 4

fig. 2

fig. 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,P | EP-A-0 416 353  (LUCAS)<br>* column 1, line 1 - line 12 *<br>* column 1, line 44 - line 55 *<br>* column 4, line 7 - line 22 *<br>* column 4, line 52 - column 5, line 29 *<br>* column 6, line 12 - line 24; figures 1-4 * *<br>– – – | 1 | A 61 M 5/32 |
| A | EP-A-0 287 950  (VENTURINI)<br>* abstract; figures 10-14 * *<br>– – – | 1 | |
| A | WO-A-8 900 435  (GAARDE)<br>* page 10, line 10 - page 26; figures 1,2 * *<br>– – – – – | 1 | |
| | | | **TECHNICAL FIELDS SEARCHED (Int. Cl.5)**<br><br>A 61 M |

The present search report has been drawn up for all claims

| Place of search | Date of completion of search | Examiner |
|---|---|---|
| The Hague | 19 December 91 | SEDY, R. |